# EUROPEAN PATENT APPLICATION

(11) **EP 4 539 191 A1**
(43) Date of publication of application: **16.04.2025**
(21) Application number: 22945339.4
(22) Date of filing: 10.06.2022
(51) Int. Cl.: H01M 10/0567, H01M 10/0525

(54) **ELECTROLYTE AND ELECTROCHEMICAL DEVICE**

(71) Applicant: Ningde Amperex Technology Limited, Ningde, Fujian 352100 (CN)
(72) Inventor: PENG, Xiexue, Ningde, Fujian 352100 (CN); JIAN, Junhua, Ningde, Fujian 352100 (CN); TANG, Chao, Ningde, Fujian 352100 (CN)
(74) Representative: Icosa
(86) International application number: PCT/CN2022/098167
(87) International publication number: WO 2023/236198

(57) **Abstract**

This application provides an electrolyte solution and an electrochemical device. The electrolyte solution includes a compound represented by Formula I: 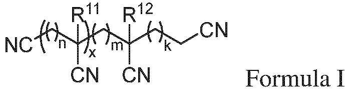 where m, n, k, and x are each independently selected from 1, 2, or 3; R¹¹ and R¹² are each independently selected from hydrogen, halogen, substituted or unsubstituted C₁ to C₃ alkyl, substituted or unsubstituted C₂ to C₄ alkenyl, substituted or unsubstituted C₂ to C₄ alkynyl, or substituted or unsubstituted C₆ to C₁₀ aryl, wherein when substituted, a substituent is independently selected from halogen. The multi-cyano (-CN) compound represented by Formula I is introduced into the electrolyte solution, so that the multi-cyano group can stabilize a transition metal in a positive active material and protect a positive electrode interface. The compound represented by Formula I can also be reduced at a negative electrode to protect a negative electrode interface and play a role in suppressing continuous decomposition of the electrolyte solution and suppressing gas production. Such a compound added can significantly improve the cycle performance and the high-temperature storage performance of the electrochemical device.

## Description

### TECHNICAL FIELD

This application relates to the field of electrochemical energy storage, and in particular, to an electrolyte solution and an electrochemical device.

### BACKGROUND

With the wide use of electrochemical devices (such as a lithium-ion battery) in various electronic products, users have put forward higher requirements on the performance of the electrochemical devices, especially the cycle performance and high-temperature storage performance. Therefore, further improvements are urgently needed to meet higher use requirements of people.

### SUMMARY

An embodiment of this application provides an electrolyte solution. The electrolyte solution includes a compound represented by Formula I:

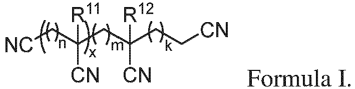

In the formula above, m, n, k, and x are each independently selected from 1, 2, or 3; R¹¹ and R¹² are each independently selected from hydrogen, halogen, substituted or unsubstituted C₁ to C₃ alkyl, substituted or unsubstituted C₂ to C₄ alkenyl, substituted or unsubstituted C₂ to C₄ alkynyl, or substituted or unsubstituted C₆ to C₁₀ aryl, where when substituted, a substituent is independently selected from halogen. A plurality of cyano groups (-CN) in the compound represented by Formula I can stabilize the high-valent transition metal in a positive electrode, and in turn, stabilize a positive electrode interface. The compound represented by Formula I can also be reduced at a negative electrode to protect a negative electrode interface and play a role in suppressing continuous decomposition of the electrolyte solution and suppressing gas production.

In some embodiments, the compound represented by Formula I includes at least one of the following compounds: 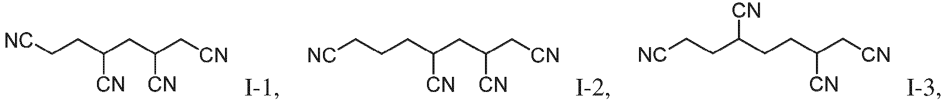 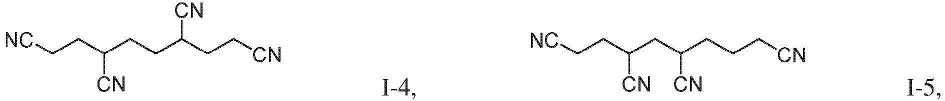 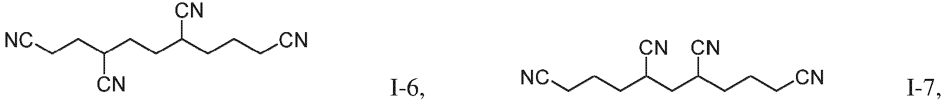 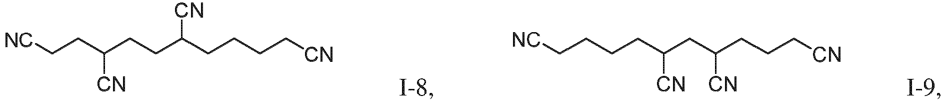 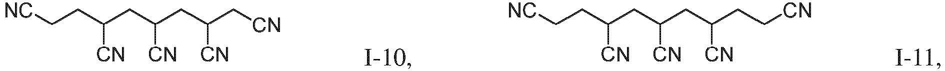 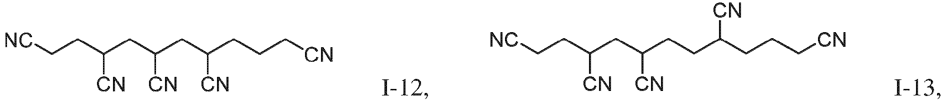 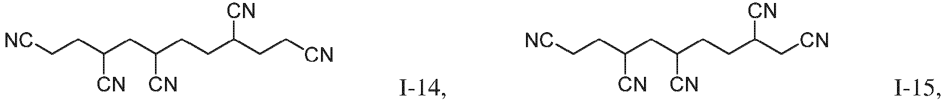 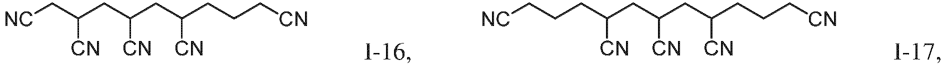 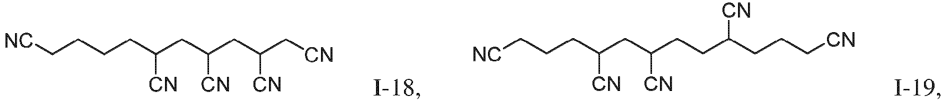 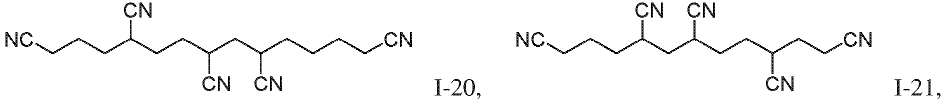 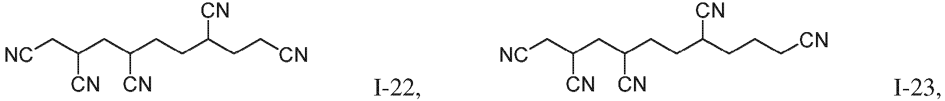 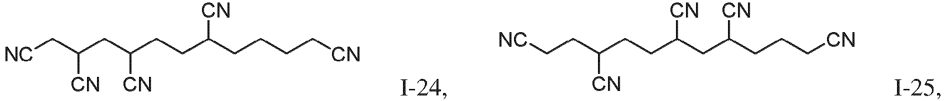 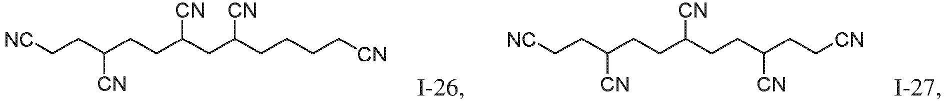 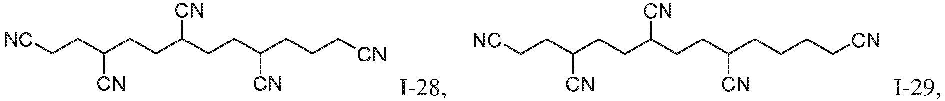 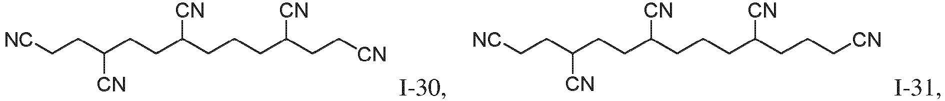 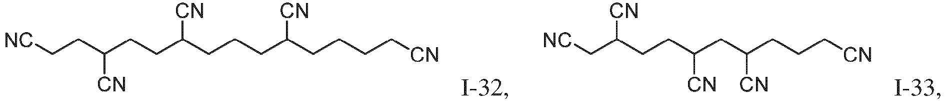 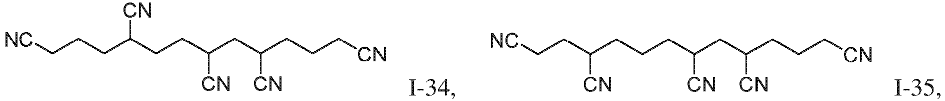 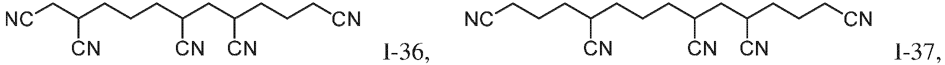 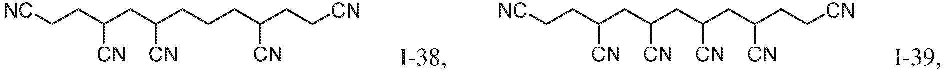 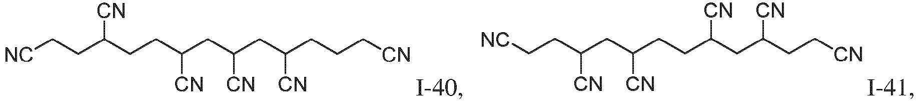 or

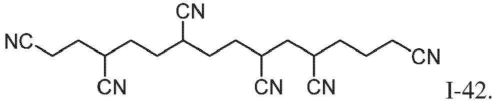

In some embodiments, based on a mass of the electrolyte solution, a mass percentage of the compound represented by Formula I is 0.01*%* to 5*%*. When the mass percentage of the compound represented by Formula I is overly low, the compound can exert just a limited effect in improving the cycle performance and high-temperature storage performance of the electrochemical device. If the mass percentage of the compound represented by Formula I is overly high, the compound makes no more significant improvement in the cycle performance and high-temperature storage performance, and the further increase of the content of the compound may cause the viscosity of the electrolyte solution to be overly high and impair the cycle performance and high-temperature storage performance of the electrochemical device. In some embodiments, based on a mass of the electrolyte solution, a mass percentage of the compound represented by Formula I is 0.1*%* to 3%.

In some embodiments, the electrolyte solution further includes a sulfur-oxygen double bond-containing compound. The sulfur-oxygen double bond-containing compound is strongly resistant to oxidation and can improve the stability of the positive electrode interface. On the other hand, the sulfur-oxygen double bond-containing compound can be reduced on the surface of the negative electrode to form a protection film to suppress the decomposition of the electrolyte solution and further enhance the stability of the negative electrode interface. In some embodiments, based on a mass of the electrolyte solution, a mass percentage of a sulfur-oxygen double bond-containing compound is 0.01*%* to 10*%*. When the mass percentage of the sulfur-oxygen double bond-containing compound is overly low, the sulfur-oxygen double bond-containing compound is not enough to protect the interface between the positive or negative electrode and the electrolyte solution, and is unable exert an effect in improving the stability of the positive and negative electrode interfaces or the effect is insignificant. When the mass percentage of the sulfur-oxygen double bond-containing compound is overly high, for example, higher than 10*%*, the compound makes no more significant improvement in the stability of the positive and negative electrode interfaces, and may result in an excessive level of the viscosity of the electrolyte solution and the impedance of the positive and negative electrode interfaces, impair the kinetics, and impair the cycle performance and high-temperature storage performance of the electrochemical device. In some embodiments, based on a mass of the electrolyte solution, a mass percentage of a sulfur-oxygen double bond-containing compound is 0.1*%* to 5*%*.

In some embodiments, the sulfur-oxygen double bond-containing compound includes the compound represented by Formula II:

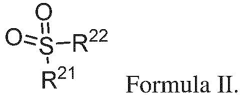

In the formula above, R²¹ and R²² are each independently selected from a substituted or unsubstituted C₁ to C₅ alkyl, a substituted or unsubstituted C₁ to C₅ alkylidene, a substituted or unsubstituted C₂ to C₁₀ alkenyl, a substituted or unsubstituted C₂ to C₁₀ alkynyl, a substituted or unsubstituted C₃ to C₁₀ alicyclyl, a substituted or unsubstituted C₆ to C₁₀ aryl, or a substituted or unsubstituted C₁ to C₆ heterocyclic group, where when substituted, a substituent is at least one of a halogen atom or a heteroatom-containing functional group, and a heteroatom in the heteroatom-containing functional group is at least one selected from B, N, O, F, Si, P, or S. In some embodiments, the sulfur-oxygen double bond-containing compound includes at least one of 1,3-propane sultone, 1,4-butane sultone, methylene methane disulfonate, 1,3-propane disulfonic anhydride, ethylene sulfate, ethylene sulfite, 4-methyl ethylene sulfate, 2,4-butane sultone, 2-methyl-1,3-propane sultone, 1,3-butane sultone, 1-fluoro-1,3-propane sultone, 2-fluoro-1,3-propane sultone, 3-fluoro-1,3-propane sultone, propenyl-1,3-sultone, propylene sulfate, propylene sulfite, or fluoroethylene sulfate.

In some embodiments, the electrolyte solution further includes a compound represented by Formula III:

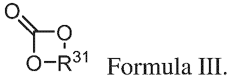

In the formula above, R³¹ is selected from a substituted or unsubstituted C₁ to C₆ alkylidene or a substituted or unsubstituted C₂ to C₆ alkenylene, where when substituted, a substituent is selected from halogen, a C₁ to C₆ alkyl, or a C₂ to C₆ alkenyl.

The compound represented by Formula III assists in enhancing the film-forming stability of the solid electrolyte interface (SEI) film of the negative electrode, increase the flexibility of the SEI film, further enhance protection for the negative active material, and reduce a probability of contact between the negative active material and the electrolyte solution interface, thereby reducing the impedance during cycling. In some embodiments, based on a mass of the electrolyte solution, a mass percentage of the compound represented by Formula III is 0.01*%* to 15*%*. If the mass percentage of the compound represented by Formula III is overly low, the compound is unable to sufficiently protect the negative electrode interface or the improvement effect is insignificant. If the mass percentage of the compound represented by Formula III is overly high, for example, higher than 15*%*, the cyclic carbonate compound makes no more significant improvement in the effect of enhancing the stability of the SEI film. In some embodiments, based on a mass of the electrolyte solution, a mass percentage of the compound represented by Formula III is 0.1*%* to 10*%*.

In some embodiments, the compound represented by Formula III includes at least one of the following compounds: 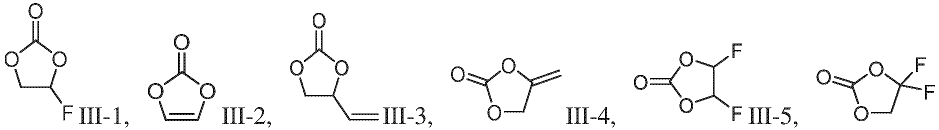 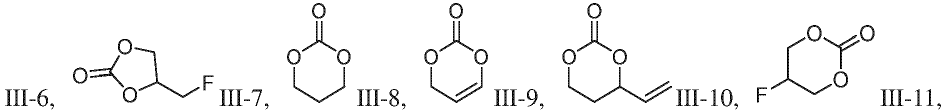 or

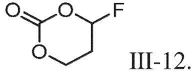

In some embodiments, the electrolyte solution further includes a polynitrile compound. The polynitrile compound can work synergistically with the compound represented by Formula I in the electrolyte solution to exert a stronger protection effect for the positive electrode interface, further suppress the decomposition of the electrolyte solution, and further improve the cycle performance and high-temperature storage performance of the electrochemical device. In some embodiments, based on a mass of the electrolyte solution, a mass percentage of the polynitrile compound is 0.01*%* to 5*%*. If the mass percentage of the polynitrile compound is overly low, the polynitrile compound is unable to exert a good effect of protecting the positive electrode interface, and the improvement effect is limited. If the mass percentage of the polynitrile compound is overly high, for example, higher than 5*%*, the polynitrile compound makes no more significant improvement in the stability of the SEI film. In some embodiments, based on a mass of the electrolyte solution, a mass percentage of the polynitrile compound is 0.1% to 3%. In some embodiments, the polynitrile compound includes at least one of the following compounds:

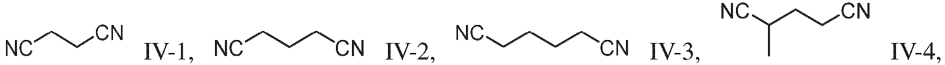

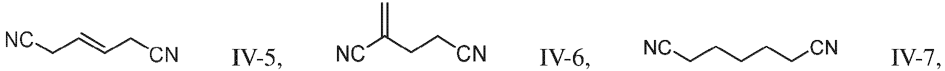

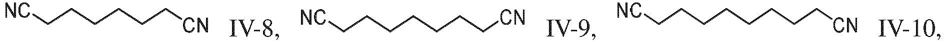

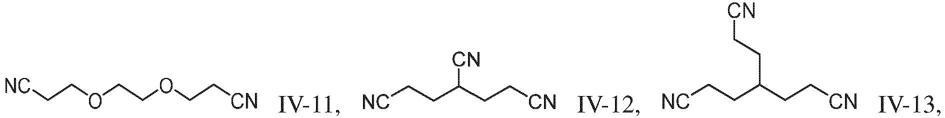

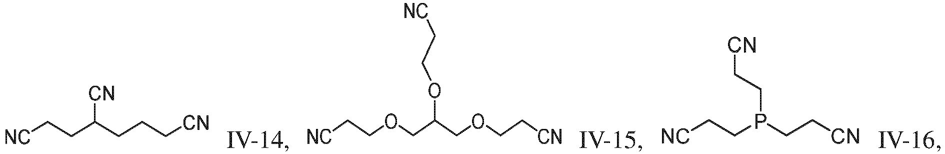

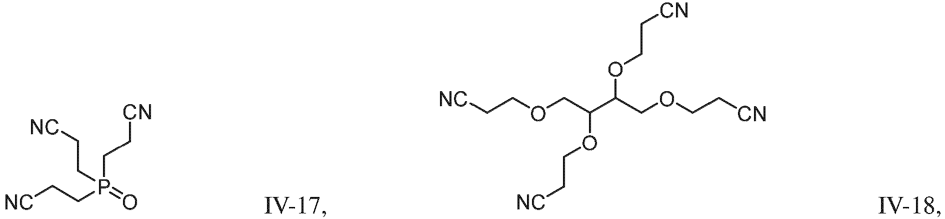

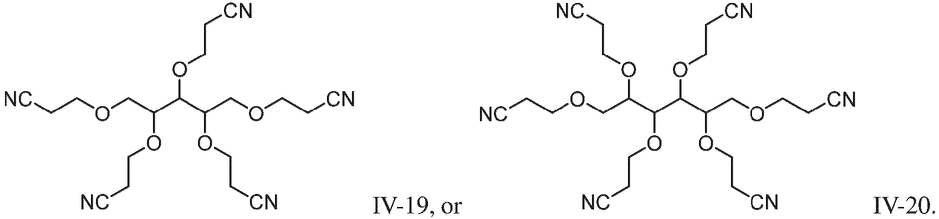

In some embodiments, the electrolyte solution further includes a boron-containing lithium salt. The boron-containing lithium salt interacts with the compound represented by Formula I. The boron-containing lithium salt can form a film at the positive electrode, and work synergistically with the compound represented by Formula I to stabilize the positive electrode interface, thereby further improving the cycle performance of the electrochemical device. In some embodiments, based on a mass of the electrolyte solution, a mass percentage of the boron-containing lithium salt is 0.01*%* to 1*%*. If the mass percentage of the boron-containing lithium salt is overly low, the boron-containing lithium salt is not enough to exert an effect of stabilizing the positive electrode interface, and can exert just a limited effect in improving the cycle performance. If the mass percentage of the boron-containing lithium salt is overly high, for example, higher than 1*%*, the boron-containing lithium salt makes no more significant improvement in the cycle performance. In some embodiments, the boron-containing lithium salt includes at least one of lithium tetrafluoroborate, lithium bis(oxalato)borate, or lithium difluoro(oxalato)borate.

In some embodiments, the electrolyte solution further includes a P-O bond-containing compound. The P-O bond-containing compound can stabilize the high-valent transition metal and oxygen atoms in the positive active material, and work synergistically with the compound represented by Formula I to protect the positive electrode interface, thereby further improving the high-temperature storage performance of the electrochemical device. In some embodiments, based on a mass of the electrolyte solution, a mass percentage of the P-O bond-containing compound is 0.01*%* to 1*%*. If the mass percentage of the P-O bond-containing compound is overly low, the P-O bond-containing compound is not enough to exert an effect of protecting the positive electrode interface, and the effect of the compound for improving the high-temperature storage performance is limited. If the mass percentage of the P-O bond-containing compound is overly high, the high-temperature storage performance of the electrochemical device will be impaired. In some embodiments, the P-O bond-containing compound includes at least one of lithium difluorophosphate, lithium difluorobis(oxalato)phosphate, lithium tetrafluoro(oxalato)phosphate, 1,2-bis((difluorophosphino)oxy)ethane, triphenyl phosphate, 3,3,3-trifluoroethyl phosphate, 3,3,3-trifluoroethyl phosphite, tris(trimethylsilane)phosphate, or 2-(2,2,2-trifluoroethoxy)-1,3,2-dioxaphosphine 2-oxide.

An embodiment of this application further provides an electrochemical device. The electrochemical device includes the above electrolyte solution.

In this application, the multi-cyano (-CN) compound represented by Formula I is introduced into the electrolyte solution, and the compound represented by Formula I contains a plurality of cyano groups. A spacing exists between the plurality of cyano groups, so that the cyano groups can strongly stabilize the transition metal in the positive active material and protect the positive electrode interface. The compound represented by Formula I can also be reduced to form a film at the negative electrode to further protect the negative electrode interface. The compound represented by Formula I can suppress decomposition of the electrolyte solution and suppress gas production, thereby significantly improving the cycle performance and high-temperature storage performance of the electrochemical device.

### DETAILED DESCRIPTION

The following embodiments enable a person skilled in the art to understand this application more comprehensively, but without limiting this application in any way.

Currently, a main method for improving the energy density of an electrochemical device includes increasing the charge voltage of the electrochemical devices. However, when the charge voltage of the electrochemical device is increased, the relatively high charge voltage accelerates the oxidative decomposition of the electrolyte solution performed by a high-valent transition metal in the positive active material. The high-valent transition metal gains electron compensation from oxygen atoms, thereby resulting in oxygen release, further accelerating the decomposition of the electrolyte solution, resulting in increased gas production of the electrochemical device, and impairing the cycle performance and high-temperature storage performance of the electrochemical device. Therefore, it is necessary to stabilize the high-valent transition metal in the positive active material to improve the cycle performance and high-temperature storage performance of the electrochemical device.

An embodiment of this application provides an electrolyte solution. The electrolyte solution includes a compound represented by Formula I:

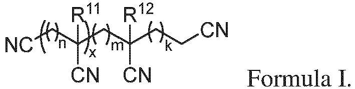

In the formula above, m, n, k, and x are each independently selected from 1, 2, or 3; R¹¹ and R¹² are each independently selected from hydrogen, halogen, substituted or unsubstituted C₁ to C₃ alkyl, substituted or unsubstituted C₂ to C₄ alkenyl, substituted or unsubstituted C₂ to C₄ alkynyl, or substituted or unsubstituted C₆ to C₁₀ aryl, where when substituted, a substituent is independently selected from halogen. The cyano group (-CN) in the compound represented by Formula I can stabilize the high-valent transition metal. The compound represented by Formula I contains a plurality of cyano groups. A specified spacing exists between the cyano groups, thereby further stabilizing the transition metal and more effectively improving the stability of the positive electrode interface. The compound represented by Formula I can also be reduced at the negative electrode to form a protection film at the negative electrode interface, thereby further protecting the negative electrode interface, suppressing the continuous decomposition of the electrolyte solution and suppressing gas production, and improving the cycle performance and high-temperature storage performance of the electrochemical device cycled at a high voltage. Therefore, the electrolyte solution can significantly improve the cycle performance and the high-temperature storage performance of the electrochemical device.

In some embodiments, the compound represented by Formula I includes at least one of the following compounds: 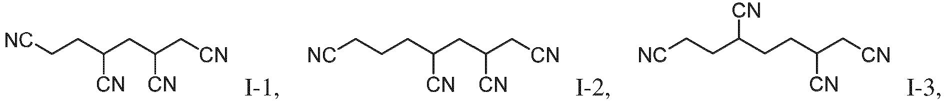 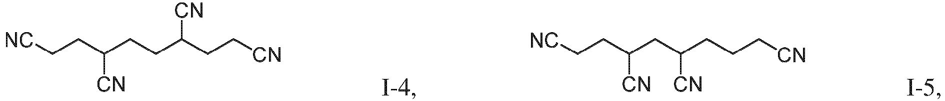 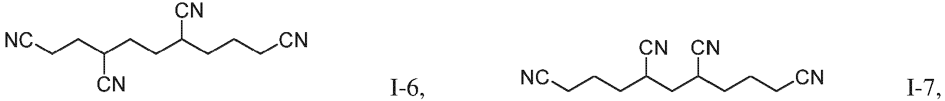 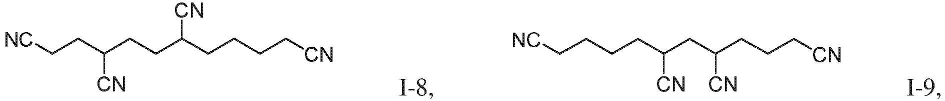 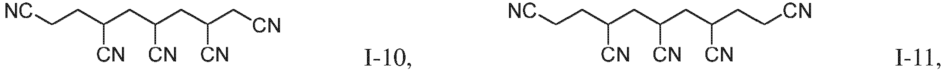 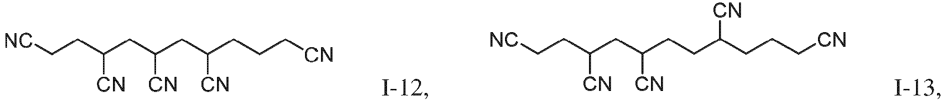 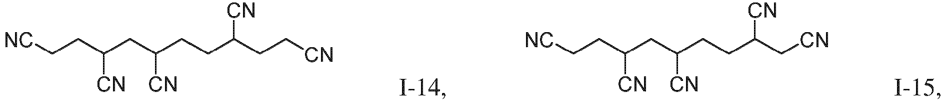 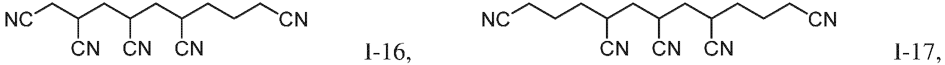 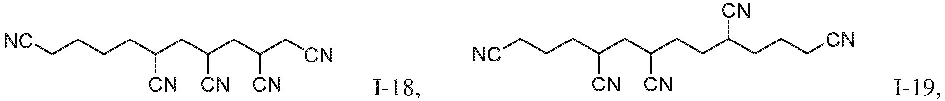 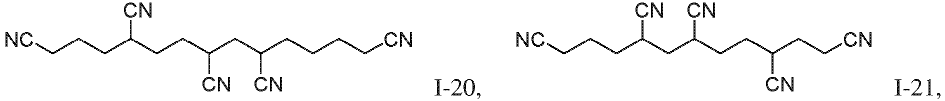 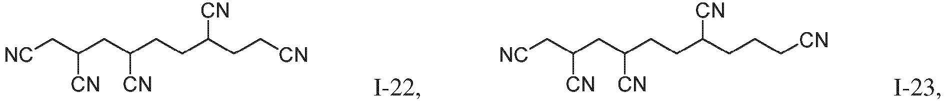 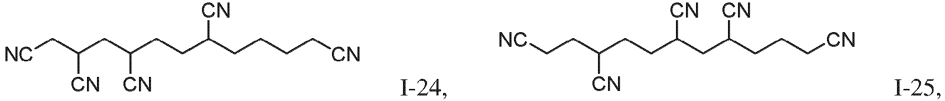 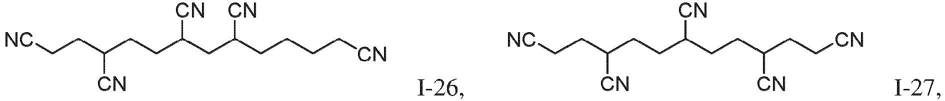 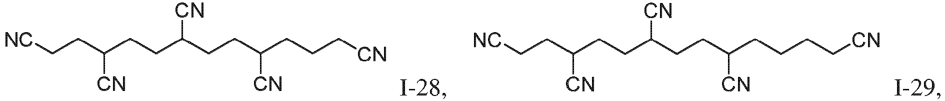 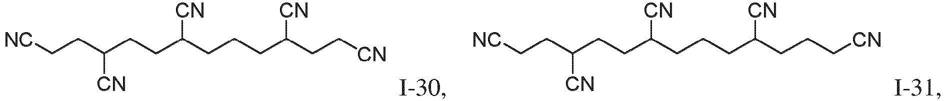 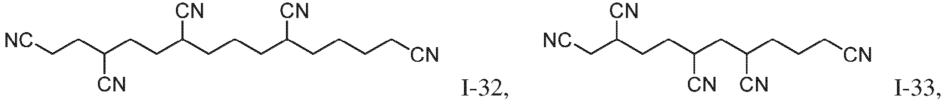 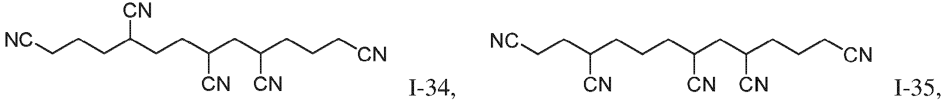 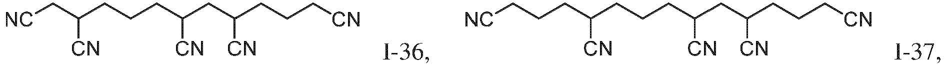 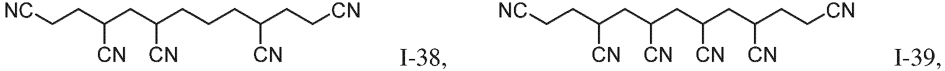 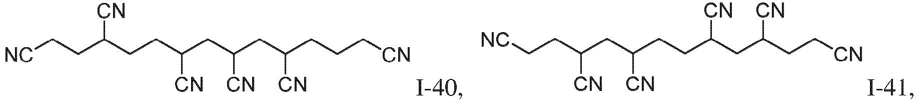 or

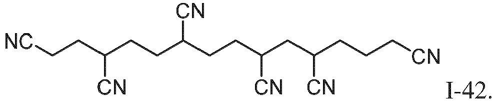

In some embodiments, based on a mass of the electrolyte solution, a mass percentage of the compound represented by Formula I is 0.01*%* to 5*%*. If the mass percentage of the compound represented by Formula I is overly low, the compound is not enough to improve the stability of the electrode interface, and therefore, can exert just a limited effect in improving the cycle performance and high-temperature storage performance of the electrochemical device. If the mass percentage of the compound represented by Formula I is overly high, the compound makes no more significant improvement in the cycle performance and high-temperature storage performance of the electrochemical device, and the further increase of the content of the compound may cause the viscosity of the electrolyte solution to be overly high and impair the interior kinetics of the electrochemical device, thereby impairing the cycle performance and high-temperature storage performance of the electrochemical device. In some embodiments, based on a mass of the electrolyte solution, a mass percentage of the compound represented by Formula I is 0.01*%*, 0.5*%*, 1*%*, 2*%*, 3*%*, 4*%*, 5*%*, or another appropriate value. In some embodiments, based on a mass of the electrolyte solution, a mass percentage of the compound represented by Formula I is 0.1*%* to 3*%*.

In some embodiments, the electrolyte solution further includes a sulfur-oxygen double bond-containing compound. The sulfur-oxygen double bond-containing compound is strongly resistant to oxidation and can improve the stability of the positive electrode interface. On the other hand, the sulfur-oxygen double bond-containing compound can be reduced on the surface of the negative electrode to form a protection film to suppress decomposition of the electrolyte solution, thereby further enhancing stability of the interface. Therefore, the sulfur-oxygen double bond-containing compound used together can further improve the cycle performance and the high-temperature storage performance of the electrochemical device. In some embodiments, the sulfur-oxygen double bond-containing compound includes the compound represented by Formula II:

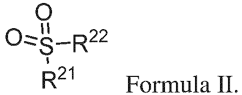

In the formula above, R²¹ and R²² are each independently selected from a substituted or unsubstituted C₁ to C₅ alkyl, a substituted or unsubstituted C₁ to C₅ alkylidene, a substituted or unsubstituted C₂ to C₁₀ alkenyl, a substituted or unsubstituted C₂ to C₁₀ alkynyl, a substituted or unsubstituted C₃ to C₁₀ alicyclyl, a substituted or unsubstituted C₆ to C₁₀ aryl, or a substituted or unsubstituted C₁ to C₆ heterocyclic group, where when substituted, a substituent is at least one of a halogen atom or a heteroatom-containing functional group, and a heteroatom in the heteroatom-containing functional group is at least one selected from B, N, O, F, Si, P, or S. In some embodiments, the sulfur-oxygen double bond-containing compound includes at least one of 1,3-propane sultone (PS), 1,4-butane sultone (BS), methylene methane disulfonate (MMDS), 1,3-propane disulfonic anhydride, ethylene sulfate, ethylene sulfite, 4-methyl ethylene sulfate, 2,4-butane sultone, 2-methyl-1,3-propane sultone, 1,3-butane sultone, 1-fluoro-1,3-propane sultone, 2-fluoro-1,3-propane sultone, 3-fluoro-1,3-propane sultone, propenyl-1,3-sultone, propylene sulfate, propylene sulfite, or fluoroethylene sulfate. In some embodiments, the sulfur-oxygen double bond-containing compound includes at least one of the compounds represented by the following chemical formulas:

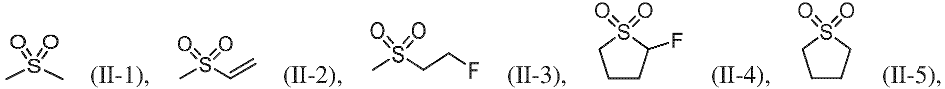

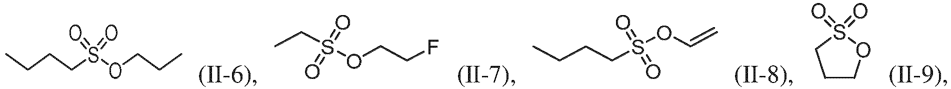

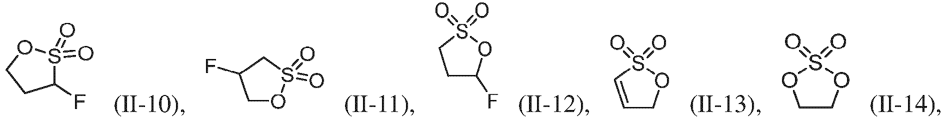

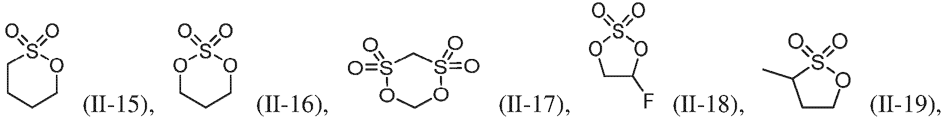

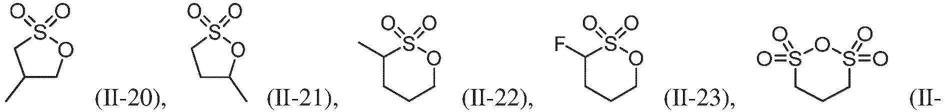

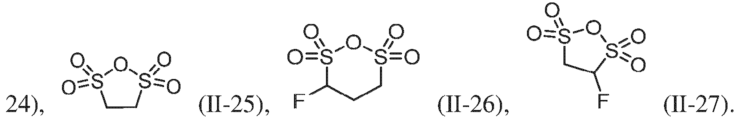

In some embodiments, based on a mass of the electrolyte solution, a mass percentage of a sulfur-oxygen double bond-containing compound is 0.01*%* to 10*%*. When the mass percentage of the sulfur-oxygen double bond-containing compound is overly low, the effect of the sulfur-oxygen double bond-containing compound for alleviating the reaction of the electrolyte solution on the positive electrode and the negative electrode is limited. If the mass percentage of the sulfur-oxygen double bond-containing compound is overly high, for example, higher than 10*%*, the sulfur-oxygen double bond-containing compound makes no more significant improvement in the stability of the positive and negative electrode interfaces, and may result in an excessive level of the viscosity of the electrolyte solution, impair the kinetics, and impair the cycle performance and high-temperature storage performance of the electrochemical device. In some embodiments, based on a mass of the electrolyte solution, a mass percentage of the *%*,sulfur-oxygen double bond-containing compound is 0.01*%*, 0.5*%*, 1*%*, 2*%*, 3*%*, 4*%*, 5*%*, 8*%*, 10*%*, or another appropriate value. In some embodiments, based on a mass of the electrolyte solution, a mass percentage of a sulfur-oxygen double bond-containing compound is 0.1*%* to 5*%*.

In some embodiments, the electrolyte solution further includes a compound represented by Formula III:

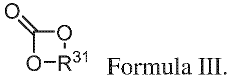

In the formula above, R³¹ is selected from a substituted or unsubstituted C₁ to C₆ alkylidene or a substituted or unsubstituted C₂ to C₆ alkenylene, where when substituted, a substituent is selected from halogen, a C₁ to C₆ alkyl, or a C₂ to C₆ alkenyl. The compound represented by Formula III can assist in enhancing the film-forming stability of the solid interface (SEI) film of the negative electrode. The compound represented by Formula III increases flexibility of the SEI film, further enhances protection for the active material, reduces the probability of contact between the active material and the electrolyte solution on the interface, and reduces the side reactions between the electrolyte solution and the active material, thereby reducing the impedance generated by accumulation of by-products during cycling. In some embodiments, the compound represented by Formula III includes at least one of the following compounds:

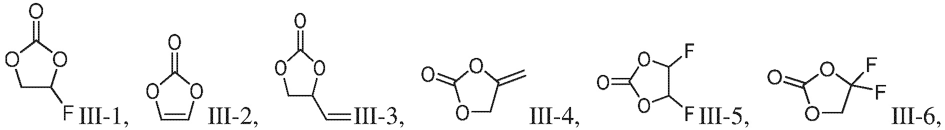

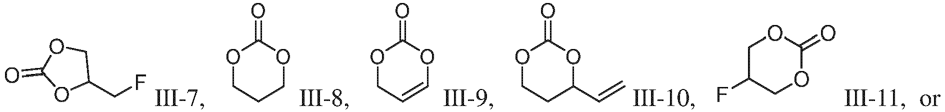

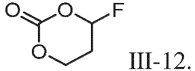

In some embodiments, based on a mass of the electrolyte solution, a mass percentage of the compound represented by Formula III is 0.01*%* to 15*%*. If the mass percentage of the compound represented by Formula III is overly low, the compound is unable to exert an effect of sufficiently protecting the interface, and improves the performance of the electrochemical device to a limited degree. If the mass percentage of the compound represented by Formula III is overly high, for example, higher than 15*%*, the cyclic carbonate compound makes no more significant improvement in the effect of enhancing the stability of the SEI film. In some embodiments, based on a mass of the electrolyte solution, a mass percentage of the compound represented by Formula III is 0.01*%*, 0.5*%*, 1*%*, 2*%*, 3*%*, 4*%*, 5*%*, 8*%*, 10*%*, 12*%*, 15*%*, or another appropriate value. In some embodiments, based on a mass of the electrolyte solution, a mass percentage of the compound represented by Formula III is 0.1*%* to 10*%*.

In some embodiments, the electrolyte solution further includes a polynitrile compound. The polynitrile compound can work synergistically with the compound represented by Formula I in the electrolyte solution to exert a stronger protection effect for the positive electrode interface, further suppress the decomposition of the electrolyte solution, and further improve the cycle performance and high-temperature storage performance of the electrochemical device. In some embodiments, the polynitrile compound includes at least one of the following compounds:

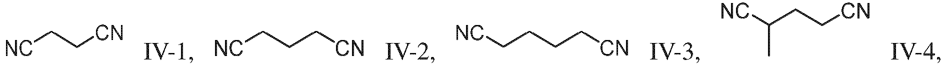

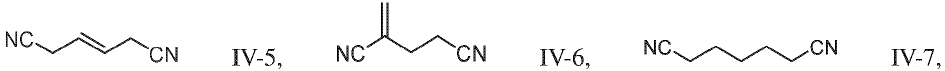

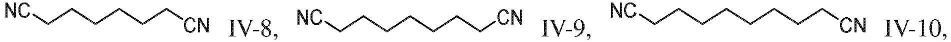

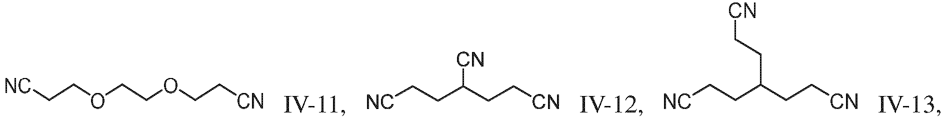

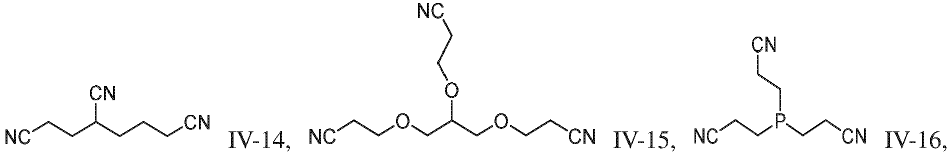

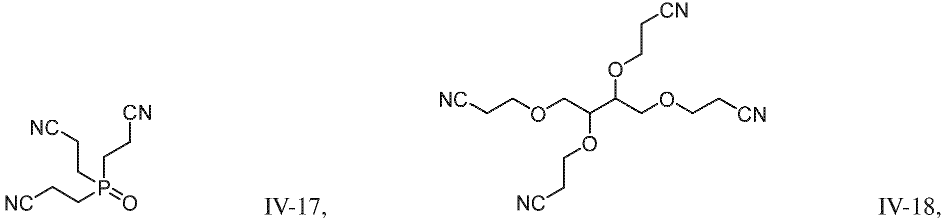

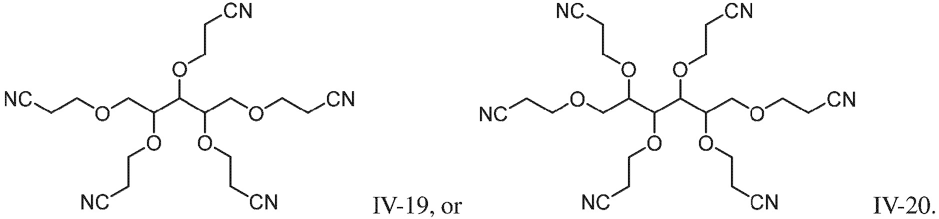

In some embodiments, based on a mass of the electrolyte solution, a mass percentage of the polynitrile compound is 0.01*%* to 5*%*. If the mass percentage of the polynitrile compound is overly low, the polynitrile compound is unable to exert a good effect of protecting the positive electrode interface, and exerts an insignificant effect in improving the performance of the electrochemical device. If the mass percentage of the polynitrile compound is overly high, for example, higher than 5*%*, the polynitrile compound makes no more significant improvement in the performance of the electrochemical device, and may result in an increased viscosity of the electrolyte solution, impair the kinetics, and impair the cycle performance and high-temperature storage performance of the electrochemical device. In some embodiments, based on a mass of the electrolyte solution, a mass percentage of the polynitrile compound is 0.01*%*, 0.5*%*, 1*%*, 2*%*, 3*%*, 4*%*, 5*%*, or another appropriate value. In some embodiments, based on a mass of the electrolyte solution, a mass percentage of the polynitrile compound is 0.1*%* to 3*%*. In some embodiments, the electrolyte solution further includes a boron-containing lithium salt. The boron-containing lithium salt can form a film at the positive electrode interface to protect the positive electrode interface, and work synergistically with the compound represented by Formula I to further improve the cycle performance of the electrochemical device. In some embodiments, the boron-containing lithium salt includes at least one of lithium tetrafluoroborate (LiBF₄), lithium bis(oxalato)borate (LiBOB), or lithium difluoro(oxalato)borate (LiDFOB). In some embodiments, based on a mass of the electrolyte solution, a mass percentage of the boron-containing lithium salt is 0.01*%* to 1*%*. If the mass percentage of the boron-containing lithium salt is overly low, the boron-containing lithium salt is not enough to protect the positive electrode interface, and can exert just a limited effect in improving the cycle performance. If the mass percentage of the boron-containing lithium salt is overly high, for example, higher than 1*%*, the boron-containing lithium salt makes no more significant improvement in the cycle performance. In some embodiments, based on a mass of the electrolyte solution, a mass percentage of the boron-containing lithium salt is 0.01*%*, 0.5*%*, 1*%*, or another appropriate value.

In some embodiments, the electrolyte solution further includes a P-O bond-containing compound. The P-O bond-containing compound can stabilize the high-valent transition metal and oxygen atoms in the positive active material to stabilize the positive electrode interface, and work synergistically with the compound represented by Formula I, thereby further improving the high-temperature storage performance of the electrochemical device. In some embodiments, the P-O bond-containing compound includes at least one of lithium difluorophosphate (LiPO₂F₂), lithium difluorobis(oxalato)phosphate (LiDFOP), lithium tetrafluoro(oxalato)phosphate (LiTFOP), 1,2-bis((difluorophosphino)oxy)ethane, triphenyl phosphate, 3,3,3-trifluoroethyl phosphate, 3,3,3-trifluoroethyl phosphite, tris(trimethylsilane)phosphate, or 2-(2,2,2-trifluoroethoxy)-1,3,2-dioxaphosphine 2-oxide. In some embodiments, based on a mass of the electrolyte solution, a mass percentage of the P-O bond-containing compound is 0.01*%* to 1*%*. If the mass percentage of the P-O bond-containing compound is overly low, the P-O bond-containing compound is not enough to exert an effect of stabilizing the electrode interface, and the effect of the compound for improving the high-temperature storage performance is limited. If the mass percentage of the P-O bond-containing compound is overly high, for example, higher than 1*%*, the P-O bond-containing compound makes no more significant improvement in the high-temperature storage performance.

In some embodiments, the electrolyte solution may further include a nonaqueous organic solvent and an electrolyte salt. The nonaqueous organic solvent may be a carbonate solvent, a carboxylate solvent, an ether solvent, or other aprotic solvents. Examples of the carbonate solvent include dimethyl carbonate, diethyl carbonate, ethyl methyl carbonate, methyl propyl carbonate, ethyl propyl carbonate, dipropyl carbonate, ethylene carbonate, propylene carbonate, butylene carbonate, bis(2,2,2-trifluoroethyl) carbonate, or the like. Examples of the carboxylate solvent include methyl acetate, ethyl acetate, n-propyl acetate, n-butyl acetate, methyl propionate, ethyl propionate, propyl propionate, butyl propionate, methyl butyrate, ethyl butyrate, propyl butyrate, butyl butyrate, γ-butyrolactone, 2,2-difluoroethyl acetate, ethyl trifluoroacetate, 2,2,3,3,3-ethyl pentafluoropropionate, 2,2,3,3,4,4,4,4-methyl heptafluorobutyrate, 4,4,4-trifluoro-3-(trifluoromethyl) methyl butyrate, 2,2,3,3,4,4,5,5,5,5-ethyl nonafluorovalerate, 2,2,3,3,4,4,5,5,6,6,7,7,8,8,9,9,9-methyl heptadecafluorononanoate, 2,2,3,3,4,4,5,5,6,6,7,7,8,8,9,9,9-ethyl heptadecafluorononanoate, and the like. Examples of the ether solvent include monoglyme, diglyme, tetraglyme, dibutyl ether, tetrahydrofuran, 2-methyltetrahydrofuran, bis(2,2,2-trifluoroethyl)ether, and the like.

In some embodiments, the electrolyte salt in this application includes at least one of an organic lithium salt or an inorganic lithium salt. In some embodiments, the electrolyte salt includes at least one of lithium hexafluorophosphate LiPF₆, lithium bis(trifluoromethanesulfonyl)imide LiN(CF₃SO₂)₂ (LiTFSI for short), lithium bis(fluorosulfonyl)imide Li(N(SO₂F)₂) (LiFSI for short), lithium hexafluorocesium oxide (LiCsF₆), lithium perchlorate LiClO₄, or lithium trifluoromethanesulfonate LiCF₃SO₃.

In some embodiments, based on a mass of the electrolyte solution, a mass percentage of the electrolyte salt is 10*%* to 15*%*. If the concentration of the electrolyte salt is overly low, the ionic conductivity of the electrolyte solution is low, thereby impairing the C-rate and cycle performance of the electrochemical device. If the concentration of the electrolyte salt is overly high, the viscosity of the electrolyte solution is overly high, thereby impairing the C-rate performance of the electrochemical device. Optionally, the mass percentage of the electrolyte salt is 12*%* to 15*%*.

An embodiment of this application further provides an electrochemical device. The electrochemical device includes an electrode assembly. The electrode assembly includes a positive electrode, a negative electrode, a separator disposed between the positive electrode and the negative electrode, and an electrolyte solution. In some embodiments, the electrolyte solution is the electrolyte solution described above.

In some embodiments, the negative electrode may include a negative current collector and a negative active material layer disposed on the negative current collector. The negative active material layer may be disposed on one side or both sides of the negative current collector. In some embodiments, the negative current collector may be at least one of a copper foil, an aluminum foil, a nickel foil, or a carbon-based current collector. In some embodiments, the thickness of the negative current collector may be 1 µm to 200 µm. In some embodiments, the negative active material layer may be applied onto just a partial region of the negative current collector. In some embodiments, the thickness of the negative active material layer may be 10 µm to 500 µm. Understandably, the examples above are merely illustrative, and other thicknesses may apply as appropriate.

In some embodiments, the negative active material layer includes a negative active material. In some embodiments, the negative active material in the negative active material layer includes at least one of a lithium metal, natural graphite, artificial graphite, or a silicon-based material. In some embodiments, the silicon-based material includes at least one of silicon, a silicon oxide compound, a silicon carbide compound, or a silicon alloy.

In some embodiments, the negative active material layer may further include a conductive agent and/or a binder. The conductive agent in the negative active material layer may include at least one of carbon black, acetylene black, Ketjen black, flake graphite, graphene, carbon nanotubes, carbon fibers, or carbon nanowires. In some embodiments, the binder in the negative active material layer may include at least one of carboxymethyl cellulose (CMC), polyacrylic acid, polyacrylate salt, polyacrylate ester, polyvinylpyrrolidone, polyaniline, polyimide, polyamideimide, polysiloxane, styrene butadiene rubber, epoxy resin, polyester resin, polyurethane resin, or polyfluorene. Understandably, the materials disclosed above are merely examples, and the negative active material layer may be made of any other appropriate materials. In some embodiments, a mass ratio between the negative active material, the conductive agent, and the binder in the negative active material layer may be (80 to 99): (0.5 to 10): (0.5 to 10). Understandably, the mass ratio is merely exemplary but not intended to limit this application.

In some embodiments, the positive electrode includes a positive current collector and a positive active material layer disposed on the positive current collector. The positive active material layer may be located on one side or both sides of the positive current collector. In some embodiments, the positive current collector may be an aluminum foil, or may be another positive current collector commonly used in this field. In some embodiments, the thickness of the positive current collector may be 1 µm to 200 µm. In some embodiments, the positive active material layer may be applied on just a partial region of the positive current collector. In some embodiments, the thickness of the positive active material layer may be 10 µm to 500 µm. Understandably, the examples above are merely illustrative, and other thicknesses may apply as appropriate.

In some embodiments, the positive active material layer includes a positive active material. In some embodiments, the positive active material includes LiCoO₂, LiNiO₂, LiMn₂O₄, LiCo_{1-y}M_{y}O₂, LiNi_{1-y}M_{y}O₂, LiMn_{2-y}M_{y}O₄, LiNiₓCo_{y}Mn_{z}M_{1-x-y-z}O₂, where M is at least one selected from Fe, Co, Ni, Mn, Mg, Cu, Zn, Al, Sn, B, Ga, Cr, Sr, V, or Ti, 0 ≤ y ≤ 1, 0 ≤ x ≤ 1, 0 ≤ z ≤ 1, and x + y + z ≤ 1. In some embodiments, the positive active material may include at least one of lithium cobalt oxide, lithium manganese oxide, lithium iron phosphate, lithium manganese iron phosphate, lithium nickel cobalt manganese oxide, lithium nickel cobalt aluminum oxide, or lithium nickel manganese oxide. The above positive active material may be doped and/or coated.

In some embodiments, the positive active material layer further includes a binder and a conductive agent. In some embodiments, the binder in the positive active material layer may include at least one of polyvinylidene difluoride, poly(vinylidene fluoride-co-hexafluoropropylene), poly(styrene-co-acrylate), poly(styrene-co-butadiene), polyamide, polyacrylonitrile, polyacrylic ester, polyacrylic acid, sodium polyacrylate, sodium carboxymethyl cellulose, polyvinyl acetate, polyvinylpyrrolidone, polyvinyl ether, polymethyl methacrylate, polytetrafluoroethylene, or polyhexafluoropropylene. In some embodiments, the conductive agent in the positive active material layer may include at least one of conductive carbon black, acetylene black, Ketjen black, flake graphite, graphene, carbon nanotubes, or carbon fibers. In some embodiments, a mass ratio of the positive active material, the conductive agent, and the binder in the positive active material layer may be (70 to 98): (1 to 15): (1 to 15). Understandably, what is described above is merely an example, and the positive active material layer may adopt any other appropriate material, thickness, and mass ratio.

In some embodiments, the separator includes at least one of polyethylene, polypropylene, polyvinylidene fluoride, polyethylene terephthalate, polyimide, or aramid fiber. For example, the polyethylene includes at least one of high-density polyethylene, low-density polyethylene, or ultra-high-molecular-weight polyethylene. Especially, the polyethylene and the polypropylene are highly effective in preventing short circuits, and can improve stability of the battery through a turn-off effect. In some embodiments, the thickness of the separator falls within a range of approximately 3 µm to 500 µm.

In some embodiments, a surface of the separator may further include a porous layer. The porous layer is disposed on at least one surface of the separator. The porous layer includes at least one of inorganic particles or a binder. The inorganic particles are at least one selected from aluminum oxide (Al₂O₃), silicon oxide (SiO₂), magnesium oxide (MgO), titanium oxide (TiO₂), hafnium dioxide (HfO₂), tin oxide (SnO₂), ceria (CeO₂), nickel oxide (NiO), zinc oxide (ZnO), calcium oxide (CaO), zirconium oxide (ZrO₂), yttrium oxide (Y₂O₃), silicon carbide (SiC), boehmite, aluminum hydroxide, magnesium hydroxide, calcium hydroxide, and barium sulfate. In some embodiments, a diameter of a pore of the separator is within a range of approximately 0.01 µm to 1 µm. The binder in the porous layer is at least one selected from polyvinylidene difluoride, poly(vinylidene difluoride-co-hexafluoropropylene), polyamide, polyacrylonitrile, polyacrylic ester, polyacrylic acid, sodium polyacrylate, sodium carboxymethyl cellulose, polyvinylpyrrolidone, polyvinyl ether, polymethyl methacrylate, polytetrafluoroethylene, and polyhexafluoropropylene. The porous layer on the surface of the separator can improve heat resistance, oxidation resistance, and electrolyte infiltration performance of the separator, and enhance adhesion between the separator and the electrode plate.

In some embodiments of this application, the electrode assembly of the electrochemical device is a jelly-roll electrode assembly or a stacked electrode assembly. In some embodiments of this application, the electrochemical device is, but not limited to, a lithium-ion battery.

In some embodiments of this application, using a lithium-ion battery as an example, the lithium-ion battery is prepared by: winding or stacking the positive electrode, the separator, and the negative electrode sequentially to form an electrode assembly, putting the electrode assembly into a package such as an aluminum laminated film housing ready for sealing, injecting an electrolyte solution, and performing chemical formation and sealing; Subsequently, a performance test is performed on the prepared lithium-ion battery.

A person skilled in the art understands that the method for preparing the electrochemical device (such as a lithium-ion battery) described above is merely an example. To the extent not departing from the content disclosed herein, other methods commonly used in the art may be employed.

An embodiment of this application further provides an electronic device containing the electrochemical device. The electronic device according to this embodiment of this application is not particularly limited, and may be any electronic device known in the prior art. In some embodiments, the electronic device may include, but not limited to, a notebook computer, pen-inputting computer, mobile computer, e-book player, portable phone, portable fax machine, portable photocopier, portable printer, stereo headset, video recorder, liquid crystal display television set, handheld cleaner, portable CD player, mini CD-ROM, transceiver, electronic notepad, calculator, memory card, portable voice recorder, radio, backup power supply, motor, automobile, motorcycle, power-assisted bicycle, bicycle, lighting appliance, toy, game console, watch, electric tool, flashlight, camera, large household battery, lithium-ion capacitor, and the like.

Some specific embodiments and comparative embodiments are enumerated below to give a clearer description of this application, using a lithium-ion battery as an example.

### Preparing a lithium-ion battery

Preparing a positive electrode: Dissolving lithium cobalt oxide (LiCoO₂) as a positive active material, conductive carbon black as a conductive agent, and polyvinylidene difluoride (PVDF) as a binder at a mass ratio of 97.9: 0.9: 1.2 in an N-methyl-pyrrolidone (NMP) solvent to form a positive electrode slurry. Using a 13 µm-thick aluminum foil as a positive current collector, applying the positive electrode slurry onto the positive current collector, and performing steps of drying, cold pressing, and cutting to obtain a positive electrode. The compaction density of the positive electrode is 4.15 g/cm³.

Preparing a negative electrode: Dissolving artificial graphite as a negative active material, styrene-butadiene rubber (SBR) as a binder, and sodium carboxymethyl cellulose (CMC) as a thickener at a mass ratio of 97.4: 1.4: 1.2 in deionized water to form a negative electrode slurry. Using a 10 µm-thick copper foil as a negative current collector, coating the negative current collector with the negative electrode slurry, and performing drying, cold pressing, and cutting to obtain a negative electrode. The compaction density of the negative electrode is 1.8 g/cm³.

Preparing a separator: Using a 5 µm-thick polyethylene (PE) film as a separator substrate, coating one side of the separator substrate with a 2 µm-thick aluminum oxide ceramic layer, and finally, applying polyvinylidene fluoride (PVDF) as a binder at a concentration of 2.5 mg/cm² onto both sides of the separator, both sides having been coated with a single ceramic layer. Performing drying to obtain a separator. The porosity of the separator is 39*%*.

Preparing an electrolyte solution: Mixing well ethylene carbonate (EC for short), propylene carbonate (PC for short), diethyl carbonate (DEC for short), ethyl propionate (EP for short), and propyl propionate (PP for short) at a mass ratio of 1: 1: 1: 1: 1 in an environment in which the water content is less than 10 ppm, and then dissolving an electrolyte salt LiPF₆ in the above nonaqueous solvent, and mixing well to form an electrolyte solution. Based on the mass of the electrolyte solution, the mass percentage of the LiPF₆ is 12.5*%*. Subsequently, adding a specified amount of additives into the electrolyte solution to obtain the electrolyte solution in each embodiment. The embodiments differ in the type and content of additives used in the electrolyte solution. The specific type of the additives and the mass percentage of the additives in the electrolyte solution are shown in Table 1 to Table 3 below. The content of each additive is a mass percentage calculated based on the mass of the electrolyte solution. The abbreviations of some of the additives are as follows: succinonitrile (SN), adiponitrile (ADN), 1,4-dicyano-2-butene (HEDN), 1,2-bis(2-cyanoethoxy)ethane (DENE), 1,3,6-hexanetrinitrile (HTCN), 1,2,3-tris(2-cyanoethoxy)propane (TCEP), 3,3',3''-phosphonitrile tripropionitrile (PTPN), 3,3',3''-(oxy-phosphonitrile)tripropionitrile (POTPN), 1,2,3,4-tetra(β-cyanoethoxy)butane (TCEB), 1,2,3,4,5-penta(β-cyanoethoxy)pentane (PCEP), and 1,2,3,4,5,6-hexa(β-cyanoethoxy)hexane (HCEH).

Preparing a lithium-ion battery: Stacking the positive electrode, the separator, and the negative electrode sequentially so that the separator is located between the positive electrode and the negative electrode to serve a function of separation, and winding the stacked plates to obtain an electrode assembly. Putting the electrode assembly into an outer package made of an aluminum laminated film, dehydrating the electrode assembly at 80 °C, injecting the electrolyte solution, and sealing the package. Performing steps such as chemical formation, degassing, and edge trimming to obtain a lithium-ion battery. The following describes the test method of each parameter in this application.

### Testing the 25 °C cycle performance:

Charging the lithium-ion battery at a current of 0.7C at a temperature of 25 °C until a voltage of 4.5 V, and then charging the battery at a constant voltage of 4.5 V until a current of 0.05C. Subsequently, discharging the battery at a current of 0.7 C until a voltage of 3.0 V, and then subjecting the battery to 800 cycles. In each cycle, the battery is charged at 0.7 C and discharged at 1 C. Using the 3^{rd}-cycle discharge capacity as a reference, using the cycle capacity retention rate as a cycle performance metric of the lithium-ion battery, and calculating the cycle capacity retention rate as:
cycle capacity retention rate = (800th-cycle discharge capacity/3rd-cycle discharge capacity) × 100%.

### Testing the high-temperature storage performance:

Charging the lithium-ion battery at a constant current of 0.5C at a temperature of 25 °C until a voltage of 4.5 V, and then charging the battery at a constant voltage until a current of 0.05C. Measuring the thickness of the lithium-ion battery, denoted as d₀. Placing the battery into an 85 °C oven to stand for 6 hours, and measuring the thickness at the end of the standing period, denoted as d. Thickness expansion rate of the lithium-ion battery after 6 hours of high-temperature storage (*%*) = (d - d₀)/d₀ × 100*%*. The test stops as soon as the thickness expansion rate exceeds 50*%*.

Table 1 shows parameters and evaluation results in Comparative Embodiment 1 and Embodiments 1 to 23.

**Table 1**

| Embodiment | Compound represented by Formula I | | Sulfur-oxygen double bond-containing compound | | | Cycle capacity retention rate | Thickness expansion rate |
|---|---|---|---|---|---|---|---|
| | Structural formula | wt*%* | II-9 | II-14 | II-19 | | |
| | | | wt*%* | wt*%* | wt*%* | | |
| Embodiment 1 | I-5 | 0.1 | | | | 61.2*%* | 35.6*%* |
| Embodiment 2 | I-5 | 0.5 | | | | 63.5*%* | 30.2*%* |
| Embodiment 3 | I-5 | 1 | | | | 66.9*%* | 25.5*%* |
| Embodiment 4 | I-5 | 2 | | | | 69.3*%* | 22.3*%* |
| Embodiment 5 | I-5 | 3 | | | | 69.6*%* | 17.9*%* |
| Embodiment 6 | I-5 | 5 | | | | 67.2*%* | 15.0*%* |
| Embodiment 7 | I-7 | 2 | | | | 68.7*%* | 22.7*%* |
| Embodiment 8 | I-17 | 2 | | | | 70.5*%* | 21.8*%* |
| Embodiment 9 | I-20 | 2 | | | | 68.8*%* | 22.5*%* |
| Embodiment 10 | I-42 | 2 | | | | 70.6*%* | 23.6*%* |
| Embodiment 11 | I-5 | 1.5 | | | | 69.5*%* | 22.5*%* |
| | I-7 | 0.5 | | | | | |
| Embodiment 12 | I-5 | 1.5 | | | | 70.3*%* | 22.7*%* |
| | I-42 | 0.5 | | | | | |
| Embodiment 13 | I-5 | 1 | | | | 70.8*%* | 21.9*%* |
| | I-17 | 0.5 | | | | | |
| | I-42 | 0.5 | | | | | |
| Embodiment 14 | I-5 | 2 | 0.1 | | | 69.4*%* | 22.0*%* |
| Embodiment 15 | I-5 | 2 | 1 | | | 69.6*%* | 20.9*%* |
| Embodiment 16 | I-5 | 2 | 2 | 0.5 | | 70.1*%* | 19.3*%* |
| Embodiment 17 | I-5 | 2 | 3 | 1 | | 71.6*%* | 18.3*%* |
| Embodiment 18 | I-5 | 2 | | 1 | 3 | 71.3*%* | 18.5*%* |
| Embodiment 19 | I-5 | 2 | 4 | 0.5 | | 70.8*%* | 17.8*%* |
| Embodiment 20 | I-5 | 2 | 5 | 0.5 | | 70.5*%* | 17.2*%* |
| Embodiment 21 | I-5 | 0.3 | 7 | 0.5 | | 70.0*%* | 16.5*%* |
| Embodiment 22 | I-4 | 0.3 | 4 | 0.5 | 3.5 | 69.2*%* | 15.0*%* |
| Embodiment 23 | I-5 | 2 | 9 | 1 | | 60.2*%* | 12.5*%* |
| Comparative Embodiment 1 | | | | | | - | Greater than 50*%* |

As can be seen from the embodiments and Comparative Embodiment 1 in Table 1, the compound represented by Formula I containing a plurality of cyano groups (-CN) can improve the cycle performance and high-temperature storage performance of the lithium-ion battery. With the increase of the mass percentage of the compound, the degree of improvement is higher, and finally a balance is achieved. The cyano group (-CN) in the compound represented by Formula I can stabilize the high-valent transition metal in the positive active material. The appropriate chain length in the compound represented by Formula I enables adjustment of the spacing between the cyano groups, thereby more effectively stabilizing the transition metal in the positive active material, more effectively protecting the electrode interface, suppressing the consumption of the electrolyte solution, and in turn, improving the cycle performance and high-temperature storage performance of the lithium-ion battery. After the sulfur-oxygen double bond-containing compound is added, the high-temperature storage performance of the lithium-ion battery is further improved.

Table 2 shows parameters and evaluation results of Embodiment 3 and Embodiments 24 to 45.

**Table 2**

| Embodiment | Compound represented by Formula I | | Sulfur-oxygen double bond-containing compound | | Polynitrile compound | | Boron-containing lithium salt | | Cycle capacity retention rate | Thickness expansion rate |
|---|---|---|---|---|---|---|---|---|---|---|
| | Structural formula | wt% | Structural formula | wt% | Structural formula | wt*%* | LiBF₄ | LiBOB | | |
| | | | | | | | wt*%* | wt*%* | | |
| Embodiment 3 | I-5 | 1 | | | | | | | 66.9*%* | 25.5*%* |
| Embodiment 24 | I-5 | 1 | | | ADN | 0.1 | | | 67.0*%* | 25.4*%* |
| Embodiment 25 | I-5 | 1 | | | ADN | 0.5 | | | 67.2*%* | 25.0*%* |
| Embodiment 26 | I-5 | 1 | | | ADN | 2 | | | 68.7*%* | 23.6*%* |
| Embodiment 27 | I-5 | 1 | | | SN | 2 | | | 70.3*%* | 21.2*%* |
| | | | | | ADN | 2 | | | | |
| | | | | | HTCN | 1 | | | | |
| Embodiment 28 | I-5 | 1 | | | ADN | 2 | | | 69.9*%* | 21.8*%* |
| | | | | | HTCN | 1 | | | | |
| Embodiment 29 | I-5 | 1 | | | ADN | 2 | | | 70.6*%* | 20.7*%* |
| | | | | | HTCN | 1 | | | | |
| | | | | | DENE | 1 | | | | |
| Embodiment 30 | I-5 | 1 | | | ADN | 2 | | | 70.7*%* | 19.9*%* |
| | | | | | HTCN | 1 | | | | |
| | | | | | HEDN | 0.5 | | | | |
| | | | | | DENE | 1 | | | | |
| Embodiment 31 | I-5 | 1 | | | ADN | 2 | | | 70.9*%* | 20.0*%* |
| | | | | | HTCN | 1 | | | | |
| | | | | | TCEP | 1 | | | | |
| Embodiment 32 | I-5 | 1 | | | ADN | 2 | | | 70.4*%* | 21.6*%* |
| | | | | | TCEB | 1 | | | | |
| Embodiment 33 | I-5 | 1 | | | ADN | 2 | | | 70.3*%* | 21.2*%* |
| | | | | | PCEP | 1 | | | | |
| Embodiment 34 | I-5 | 1 | | | ADN | 2 | | | 70.6*%* | 21.7*%* |
| | | | | | HCEH | 1 | | | | |
| Embodiment 35 | I-5 | 1 | | | PTPN | 0.5 | | | 69.5*%* | 22.4*%* |
| | | | | | ADN | 2 | | | | |
| Embodiment 36 | I-5 | 1 | | | POPTN | 0.5 | | | 68.7*%* | 23.9*%* |
| Embodiment 37 | I-5 | 1 | | | | | | 0.1 | 67.4*%* | 25.2*%* |
| Embodiment 38 | I-5 | 1 | | | | | | 0.5 | 68.2*%* | 26.2*%* |
| Embodiment 39 | I-5 | 1 | | | | | | 0.7 | 69.6*%* | 28.5*%* |
| Embodiment 40 | I-5 | 1 | | | | | | 1 | 69.2*%* | 32.7*%* |
| Embodiment 41 | I-5 | 1 | | | | | 0.2 | | 67.0*%* | 25.1*%* |
| Embodiment 42 | I-5 | 2 | II-12 | 4 | ADN | 2 | | | 71.9*%* | 14.5*%* |
| | | | II-17 | 0.5 | HCEH | 1 | | | | |
| Embodiment 43 | I-5 | 2 | II-12 | 4 | | | 0.2 | | 71.0*%* | 17.2*%* |
| | | | II-17 | 0.5 | | | | | | |
| Embodiment 44 | I-5 | 1 | | | ADN | 2 | | 0.5 | 70.8*%* | 20.2*%* |
| | | | | | PTPN | 0.5 | | | | |
| Embodiment 45 | I-5 | 1.5 | II-12 | 4 | POPTN | 0.5 | 0.1 | 0.5 | 73.2*%* | 18.5*%* |

As can be seen from Embodiments 24 to 45 versus Embodiment 3, the combination of the compound represented by Formula I and the sulfur-oxygen double bond-containing compound can improve the high-temperature storage performance of the lithium-ion battery, the combination with a boron-containing lithium salt can improve the cycle performance of the lithium-ion battery, and the combination with a polynitrile compound can improve the high-temperature storage performance and cycle performance of the lithium-ion battery. By combining a variety of additives, the lithium-ion battery can achieve a good level of both cycle performance and high-temperature storage performance.

Table 3 shows parameters and evaluation results of Embodiment 4 and Embodiments 46 to 57.

**Table 3**

| Embodiment | Compound represented by Formula I | | Compound represented by Formula III | | P-O bond-containing compound | | Cycle capacity retention rate | Thickness expansion rate |
|---|---|---|---|---|---|---|---|---|
| | Structural formula | wt*%* | Structural formula | wt*%* | LiPO₂F₂ | LiDFOP | | |
| | | | | | wt*%* | wt*%* | | |
| Embodiment 4 | I-5 | 2 | | | | | 69.3*%* | 22.3*%* |
| Embodiment 46 | I-5 | 2 | III-1 | 0.1 | | | 69.8*%* | 21.9*%* |
| Embodiment 47 | I-5 | 2 | III-1 | 5 | | | 75.5*%* | 26.5*%* |
| Embodiment 48 | I-5 | 2 | III-1 | 10 | | | 78.2*%* | 45.3*%* |
| Embodiment 49 | I-5 | 2 | III-1 | 15 | | | 79.0*%* | Greater than 50*%* |
| Embodiment 50 | I-5 | 2 | III-1 | 30 | | | 80.3*%* | Greater than 50*%* |
| Embodiment 51 | I-5 | 2 | III-1 | 5 | | | 77.6*%* | 28.2*%* |
| | | | III-2 | 0.5 | | | | |
| Embodiment 52 | I-5 | 2 | | | 0.1 | | 69.5*%* | 21.7*%* |
| Embodiment 53 | I-5 | 2 | | | 0.3 | | 69.8*%* | 21.0*%* |
| Embodiment 54 | I-5 | 2 | | | 0.5 | | 70.5*%* | 20.2*%* |
| Embodiment 55 | I-5 | 2 | | | 1 | | 69.3*%* | 18.5*%* |
| Embodiment 56 | I-5 | 2 | | | | 0.3 | 69.9*%* | 21.2*%* |
| Embodiment 57 | I-5 | 2 | | | 0.3 | 0.2 | 70.1*%* | 19.3*%* |

As can be seen from comparison between Embodiment 4 and Embodiments 46 to 57, the combination of the compound represented by Formula I and the compound represented by Formula III can significantly improve the cycle performance of the lithium-ion battery, and can also coordinate the high-temperature storage performance. In addition, the P-O bond-containing compound further added in the electrolyte solution can further improve the high-temperature storage performance of the lithium-ion battery.

What is described above is merely exemplary embodiments of this application and the technical principles thereof. A person skilled in the art understands that the scope of disclosure in this application is not limited to the technical solutions formed by a specific combination of the foregoing technical features, but covers other technical solutions formed by arbitrarily combining the foregoing technical features or equivalents thereof, for example, a technical solution formed by replacing any of the foregoing features with a technical feature disclosed herein and serving similar functions.

## Claims

1. An electrolyte solution, comprising a compound represented by Formula I: 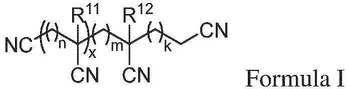 wherein m, n, k, and x are each independently selected from 1, 2, or 3; R¹¹ and R¹² are each independently selected from hydrogen, halogen, substituted or unsubstituted C₁ to C₃ alkyl, substituted or unsubstituted C₂ to C₄ alkenyl, substituted or unsubstituted C₂ to C₄ alkynyl, or substituted or unsubstituted C₆ to C₁₀ aryl, wherein when substituted, a substituent is independently selected from halogen.

2. The electrolyte solution according to claim 1, wherein the compound represented by Formula I comprises at least one of the following compounds: 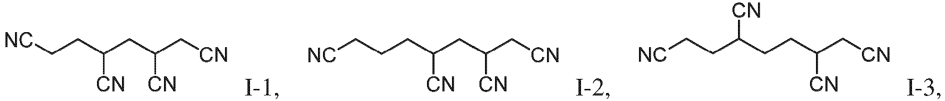 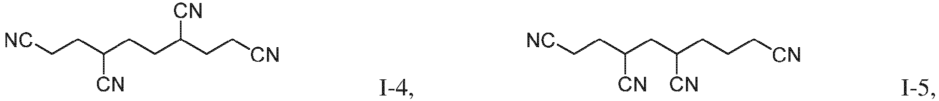 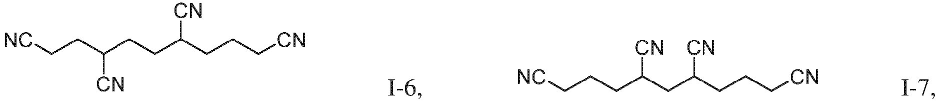 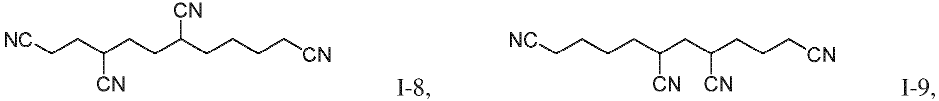 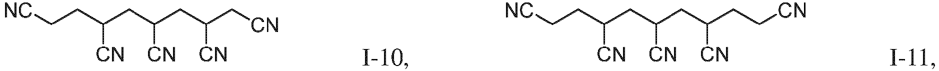 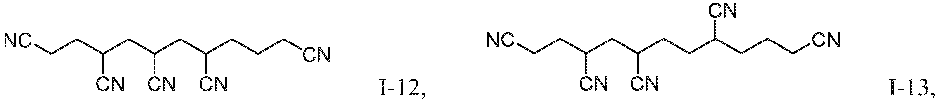 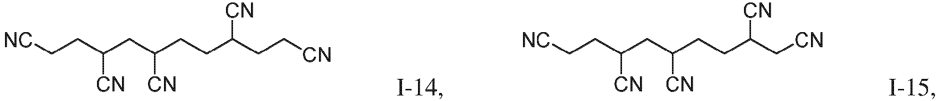 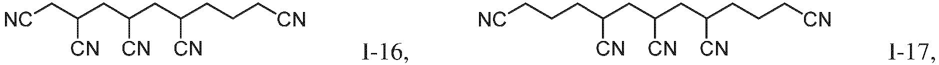 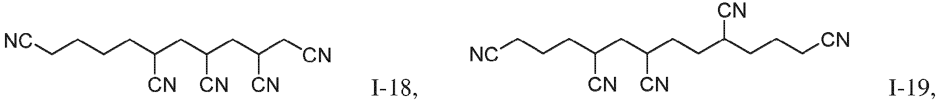 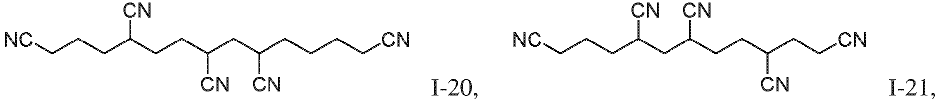 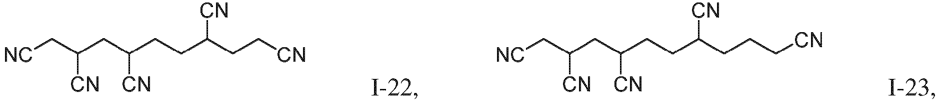 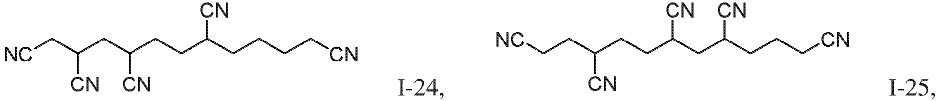 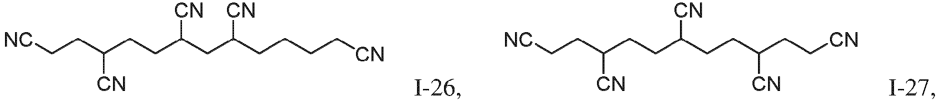 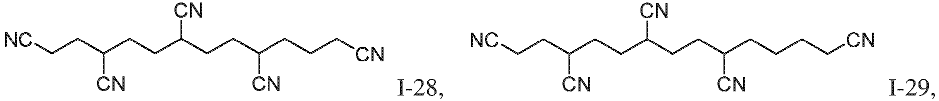 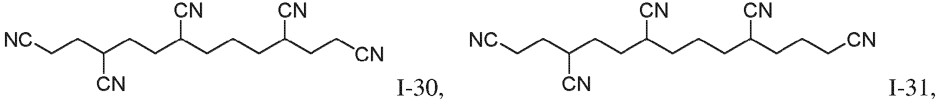 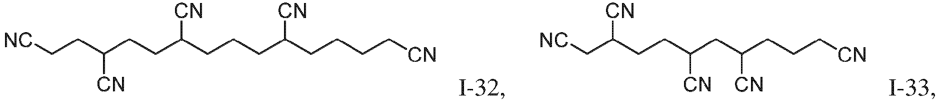 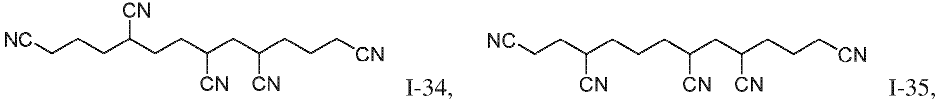 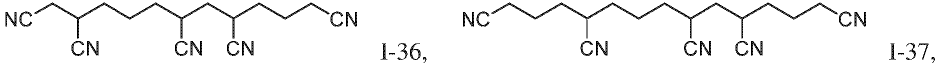 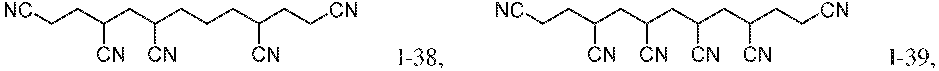 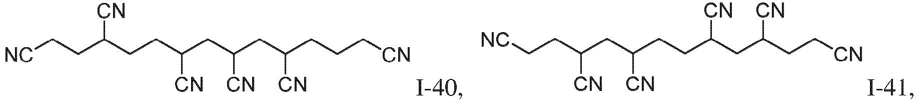 or

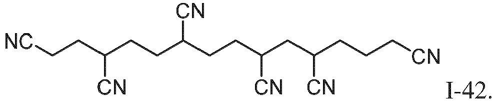

3. The electrolyte solution according to claim 1, wherein, based on a mass of the electrolyte solution, a mass percentage of the compound represented by Formula I is 0.01*%* to 5*%*.

4. The electrolyte solution according to claim 1, wherein the electrolyte solution further comprises a sulfur-oxygen double bond-containing compound; and based on a mass of the electrolyte solution, a mass percentage of the sulfur-oxygen double bond-containing compound is 0.01*%* to 10*%*.

5. The electrolyte solution according to claim 4, wherein the sulfur-oxygen double bond-containing compound comprises a compound represented by Formula II: 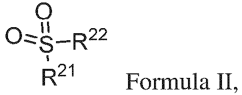 wherein R²¹ and R²² are each independently selected from a substituted or unsubstituted C₁ to C₅ alkyl, a substituted or unsubstituted C₁ to C₅ alkylidene, a substituted or unsubstituted C₂ to C₁₀ alkenyl, a substituted or unsubstituted C₂ to C₁₀ alkynyl, a substituted or unsubstituted C₃ to C₁₀ alicyclyl, a substituted or unsubstituted C₆ to C₁₀ aryl, or a substituted or unsubstituted C₁ to C₆ heterocyclic group, wherein when substituted, a substituent is at least one of a halogen atom or a heteroatom-containing functional group, and a heteroatom in the heteroatom-containing functional group is at least one selected from B, N, O, F, Si, P, or S.

6. The electrolyte solution according to claim 4, wherein the sulfur-oxygen double bond-containing compound comprises at least one of 1,3-propane sultone, 1,4-butane sultone, methylene methane disulfonate, 1,3-propane disulfonic anhydride, ethylene sulfate, ethylene sulfite, 4-methyl ethylene sulfate, 2,4-butane sultone, 2-methyl-1,3-propane sultone, 1,3-butane sultone, 1-fluoro-1,3-propane sultone, 2-fluoro-1,3-propane sultone, 3-fluoro-1,3-propane sultone, propenyl-1,3-sultone, propylene sulfate, propylene sulfite, or fluoroethylene sulfate.

7. The electrolyte solution according to claim 1, wherein the electrolyte solution further comprises a compound represented by Formula III, 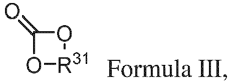
wherein R³¹ is selected from a substituted or unsubstituted C₁ to C₆ alkylidene or a substituted or unsubstituted C₂ to C₆ alkenylene, wherein when substituted, a substituent is selected from halogen, a C₁ to C₆ alkyl, or a C₂ to C₆ alkenyl; and
based on a mass of the electrolyte solution, a mass percentage of the compound represented by Formula III is 0.01*%* to 15*%*.

8. The electrolyte solution according to claim 7, wherein the compound represented by Formula III comprises at least one of the following compounds:

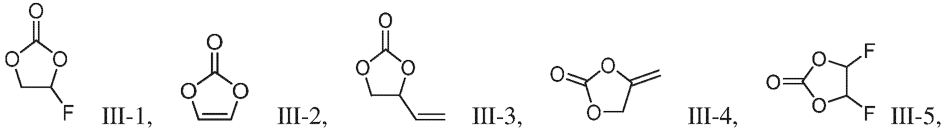

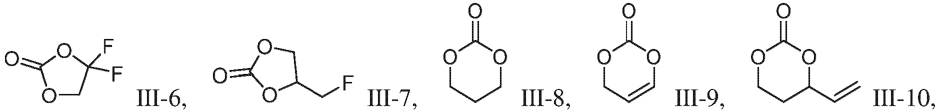

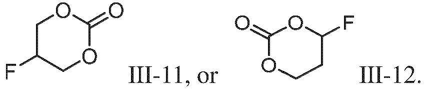

9. The electrolyte solution according to claim 1, wherein the electrolyte solution further comprises a polynitrile compound in addition to the compound represented by Formula I, and, based on a mass of the electrolyte solution, a mass percentage of the polynitrile compound is 0.01*%* to 5*%*.

10. The electrolyte solution according to claim 9, wherein the polynitrile compound comprises at least one of the following compounds:

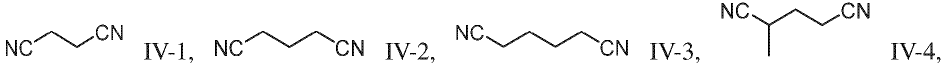

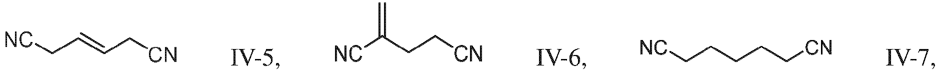

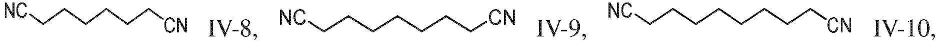

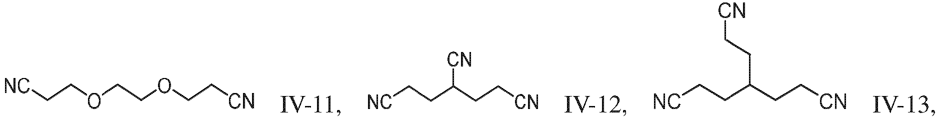

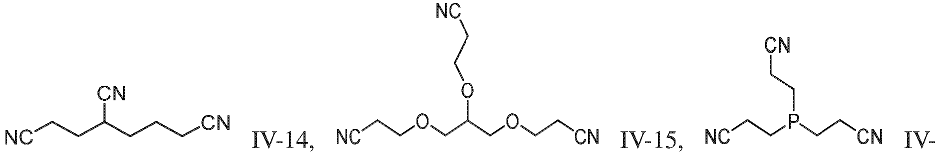

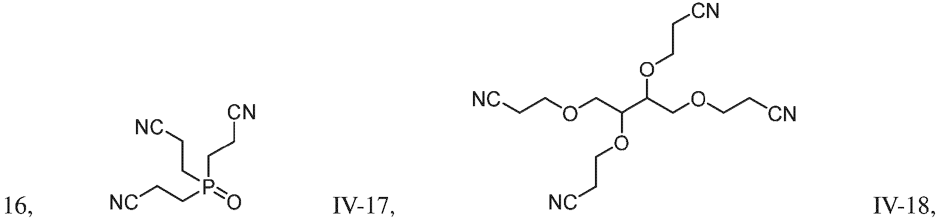

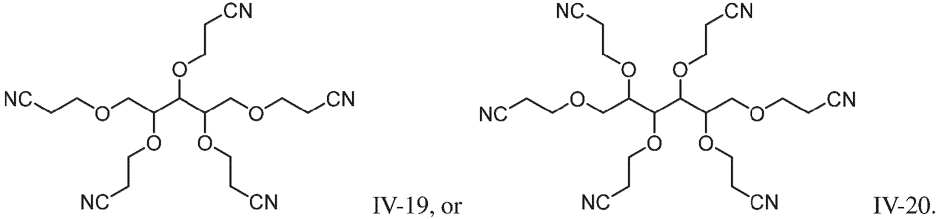

11. The electrolyte solution according to claim 1, wherein the electrolyte solution further comprises a boron-containing lithium salt, and based on a mass of the electrolyte solution, a mass percentage of the boron-containing lithium salt is 0.01*%* to 1*%*.

12. The electrolyte solution according to claim 11, wherein the boron-containing lithium salt comprises at least one of lithium tetrafluoroborate, lithium bis(oxalato)borate, or lithium difluoro(oxalato)borate.

13. The electrolyte solution according to claim 1, wherein the electrolyte solution further comprises a P-O bond-containing compound, and based on a mass of the electrolyte solution, a mass percentage of the P-O bond-containing compound is 0.01*%* to 1*%*.

14. The electrolyte solution according to claim 13, wherein the P-O bond-containing compound comprises at least one of lithium difluorophosphate, lithium difluorobis(oxalato)phosphate, lithium tetrafluoro(oxalato)phosphate, 1,2-bis((difluorophosphino)oxy)ethane, triphenyl phosphate, 3,3,3-trifluoroethyl phosphate, 3,3,3-trifluoroethyl phosphite, tris(trimethylsilane)phosphate, or 2-(2,2,2-trifluoroethoxy)-1,3,2-dioxaphosphine 2-oxide.

15. The electrolyte solution according to any one of claims 1 to 14, wherein the electrolyte solution satisfies at least one of the following conditions:
(1) based on a mass of the electrolyte solution, a mass percentage of the compound represented by Formula I is 0.1% to 3%;
(2) based on a mass of the electrolyte solution, a mass percentage of a sulfur-oxygen double bond-containing compound is 0.1% to 5%;
(3) based on a mass of the electrolyte solution, a mass percentage of a compound represented by Formula III is 0.1% to 10%; or
(4) based on a mass of the electrolyte solution, a mass percentage of a polynitrile compound is 0.1*%* to 3*%*.

16. An electrochemical device, wherein the electrochemical device comprises the electrolyte solution according to any one of claims 1 to 15.
